(19) Europäisches Patentamt
European Patent Office
Office européen des brevets

(11) EP 0 704 861 B1

(12) # FASCICULE DE BREVET EUROPEEN

(45) Date de publication et mention de la délivrance du brevet:
**07.11.2007 Bulletin 2007/45**

(51) Int Cl.:
***H01B 3/22*** *(2006.01)*

(21) Numéro de dépôt: **95402099.6**

(22) Date de dépôt: **19.09.1995**

(54) **Utilisation pour les transformateurs de distribution d'une composition diélectrique à base de polyarylalcanes ayant des propriétés diélectriques améliorées**

Verwendung für Verteilertransformatoren von einer dielektrischen Zusammensetzung, basierend auf Polyarylalkanen mit verbesserten dielektrischen Eigenschaften

Use for distribution transformers of a dielectric composition based on polyarylalkanes with improved dielectric properties

(84) Etats contractants désignés:
**AT BE CH DE DK ES FR GB GR IE IT LI LU NL PT SE**

(30) Priorité: **30.09.1994 FR 9411718**

(43) Date de publication de la demande:
**03.04.1996 Bulletin 1996/14**

(60) Demande divisionnaire:
**05077612.9 / 1 630 824**

(73) Titulaire: **ARKEMA FRANCE**
**92700 Colombes (FR)**

(72) Inventeurs:
- **Berger, Noelle**
  **F-69130 Ecully (FR)**
- **Commandeur, Raymond**
  **F-38220 Vizille (FR)**

(74) Mandataire: **Dang, Doris et al**
**ARKEMA FRANCE**
**Département Propriété Industrielle**
**420, rue d'Estienne d'Orves**
**92705 Colombes Cedex (FR)**

(56) Documents cités:
**EP-A- 0 154 993**
**EP-A- 0 172 537**

- **CHEMICAL ABSTRACTS, vol. 80, no. 24, 17 Juin 1974 Columbus, Ohio, US; abstract no. 138427c, page 361; & JP-A-04 897 096 (NIPPON PETROCHEMICAL) 11 Décembre 1973**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une composition diélectrique à base de polyarylalcanes ayant des propriétés diélectriques améliorées et son utilisation dans les appareils électriques fonctionnant à haute température, notamment les transformateurs de distribution.

Une tendance actuelle est d'utiliser des transformateurs de distribution dont on s'efforce d'augmenter la puissance tout en conservant les mêmes dimensions.

Cette augmentation de puissance volumique entraîne une augmentation des températures de fonctionnement qui peuvent passer de 60°C - 80°C pour des transformateurs classiques, à 150°C voir 200°C pour les transformateurs de distribution dits "haute température".

Pour ce nouveau type d'appareil, on recherche des liquides qui possèdent de bonnes propriétés diélectriques même à haute température, une bonne stabilité thermique, une faible tension de vapeur et une viscosité suffisamment faible pour éliminer les calories.

Les huiles isolantes couramment utilisées telles que les huiles minérales, les huiles silicones, les esters de pentaerythritol possèdent un comportement à haute température relativement médiocre. Notamment il a été observé pour les huiles silicones un abaissement de la tension de claquage lorsque la température s'élevait (Etude effectuée par General Electric Co. pour le U.S. Department of Energy - rapport HCP/T-2115 publié en février 1979 - figure 3.3. page 35).

Ce comportement semble général car il a été également observé sur des liquides de synthèse tels que l'hexane (IEEE Trans. Electr. Insul. Vol El - 13 No4, August 1978 p263) et sur des huiles minérales ("Insulating Materials for Design and Pratice" de Franck M. Clark page 151- Ed. John Wiley and Sons, Inc).

Ceci présente notamment l'inconvénient de ne pouvoir garder les mêmes distances entre les conducteurs si l'on souhaite augmenter la puissance des appareils et, par conséquent, conduit à augmenter leur taille.

Par ailleurs, les huiles minérales et les huiles silicones présentent des stabilités thermiques limitées.

En outre, les huiles silicones sont de mauvais agents de transfert de chaleur.

On a maintenant trouvé une composition qui possède de meilleurs propriétés diélectriques, notamment, une tension de claquage qui augmente avec la température, une bonne stabilité thermique, une faible tension de vapeur et qui est également un bon agent de transfert de chaleur.

La composition selon l'invention est caractérisée en ce qu'elle comprend au moins un oligomère de polyarylalcane, qui consiste en un isomère ou un mélange d'isomères de formule :

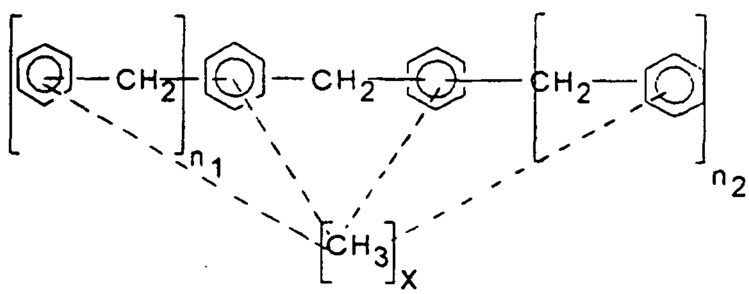

(I)

dans laquelle n1 et n2 = 0, 1 ou 2 sachant que n1 + n2 est égal à 1 ou 2
x = 0,1,2,3,4, 5 ou 6 et que ladite composition a

a) une tension de vapeur au plus égale à 1 torr à 150°C et, de préférence au plus égale à 0,25 torr,

b) un coefficient de qualité thermique défini comme le produit de la chaleur spécifique à 100°C (J/l/°C) par la conductivité thermique à 100°C (W/m/°C) divisé par la viscosité à 100°C ($mm^2/s$) et multiplié par 100 au moins égal à 3 et, de préférence au moins égal à 6,

c) un taux de formation de légers à 260°C pendant 500 heures au plus égal à 3g/kg et, de préférence, au plus égal à 1g/kg,

d) une tension de claquage à 20°C au moins égale à 60kV, et de préférence au moins égale à 80 kV, et à 80°C, au moins égal à 110 kV et, de préférence au moins égale à 130 kV mesurée en courant alternatif de $50H_z$ dans une cellule équipée d'une électrode constituée par un barreau de diamètre égal à 0,6 mm et d'une électrode constituée par un disque Rogowski à bord arrondi de diamètre égal à 40 mm, lesdites électrodes étant séparées par une distances de 40 mm.

A titre d'illustration d'oligomère de formule (I) entrant dans la composition selon l'invention on peut citer :

- l'oligomère de formule (I) dans laquelle n1 = 1, n2 = 0 et x = 1

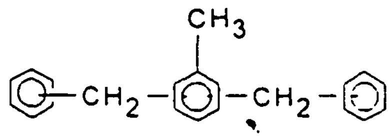

(Ia)

- l'oligomère de formule (I) dans laquelle n1 = 0, n2 = 1 et x = 1

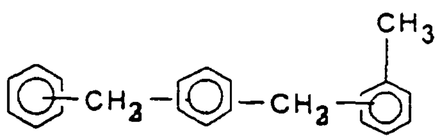

(Ib)

- l'oligomère de formule (I) dans laquelle $n_1$ et $n_2$ = 0, 1 ou 2 sachant que $n_1$ + $n_2$ = 2 et x = 1.

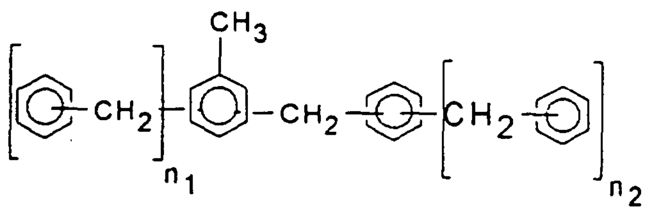

(Ic)

Selon la présente invention on préfère utiliser une composition comprenant une quantité pondérale d'oligomères (Ia) et (Ib) au moins égale à 40% et, de préférence une quantité pondérale allant de 70% à plus de 99% et une quantité pondérale d'oligomères (Ic) au plus égale à 3 % et, de préférence une quantité pondérale allant de 0,5 % à 2 %.

La composition de la présente invention peut éventuellement contenir de très faibles quantités d'oligomères de polyarylalcanes de formule (I) dans laquelle n1 = n2 = 0 et x = 1 (benzyltoluènes) voir même des oligomères de polyarylalcanes de formule (I) dans laquelle n1 = n2 = 0 et x = 0 (benzylbenzène).

Il est souhaitable que la quantité pondérale en benzyltoluènes et benzylbenzènes dans la composition soit aussi faible que possible, voir quasiment nulle car leur présence est de nature à augmenter notamment la tension de vapeur de ladite composition, ce qui est rédhibitoire pour l'utilisation envisagée.

Ces composés ont également l'inconvénient d'abaisser considérablement les points d'éclair et de feu des compositions les contenant. Ceci est rédhibitoire lorsque ces compositions doivent être utilisées dans des transformateurs de distribution haute température.

On ne sortirait pas du cadre de l'invention si l'oligomère de polyarylalcane de formule (I) était accompagné d'au moins un oligomère de polyarylalcane, consistant en un isomère ou mélange d'isomères de formule

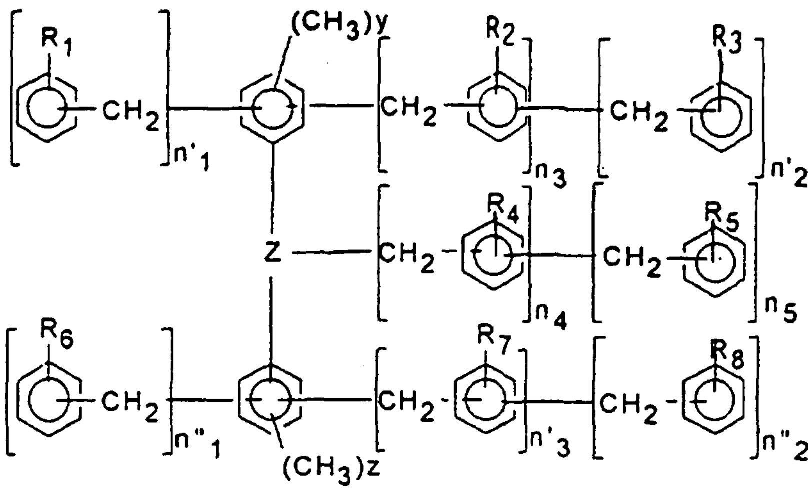

(II)

dans laquelle Z est un groupe de liaison trivalent tel que :

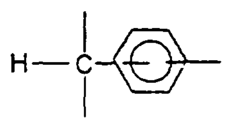

,

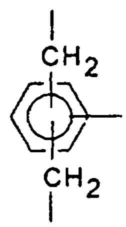

$R_1$, $R_2$, $R_3$, $R_4$, $R_5$, $R_6$, $R_7$, $R_8$ sont identiques au différents et représentent H ou $CH_3$,
n'1,n"1 et n 4 = 0,1 ou 2 ;
n'2, n"2, n3, $n'_3$ et $n_5$ = 0 ou 1 ;
n'1 + n"1 + n'2 + n"2 + n3 + n'3 + n4 + n5 ≤ 2,
y et z = 0,1 ou 2

A titre d'illustration d'oligomères de formule (II), on peut citer les ditolylphénylméthanes :

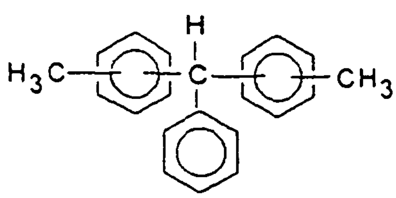

(IIa)

Selon la présente invention, la composition peut comprendre une quantité pondérale d'oligomères de formule (II) au plus égale à 10 %.

Selon une forme préférée de l'invention, la composition peut comprendre de :

. 60% à 70% en poids d'oligomères (Ia),
. 20% à 30% en poids d'oligomères (Ib),
. 0 % à 2 % en poids d'oligomères (Ic),
. 0% à 10% en poids de ditolylphenylméthanes (IIa)

Le total étant égal à 100%.

Les différents produits ou isomères de la composition selon l'invention, c'est-à-dire les oligomères de polyarylalcane de formule (I) et (II) peuvent être préparés par condensation du chlorure de benzyle ou de (méthyl)benzyle

contenant une quantité variable de chlorure de benzylidène sur le toluène ou le xylène ou le mélange des deux en présence de catalyseur de Friedel et Crafts.

La condensation a lieu en pratique à une température comprise entre 50°C et 150°C.

La quantité de catalyseur est généralement comprise entre 50 ppm et 1% en poids de la masse réactionnelle.

A titre de catalyseur de Friedel et Crafts on peut utiliser des halogénures métalliques tels que le chlorure ferrique, le chlorure d'aluminium, le trichlorure d'antimoine, le tétrachlorure de titane.

Le mélange réactionnel obtenu est constitué essentiellement d'oligomères de polyarylalcane de formule (I) et (II) et de toluène non réagi.

Le mélange réactionnel peut subir un traitement de déchloration. A cet effet, on peut utiliser le procédé décrit dans EP 306398 utilisant un alcoolate de métal alcalin ou bien le procédé décrit dans EP 225849 utilisant le sodium métal, le contenu de ces demandes étant incorporé dans la présente invention.

Après le traitement de déchloration, on effectue une simple distillation, généralement sous pression réduite pour récupérer les oligomères de formule (I) et, éventuellement (II).

Les légers tels que les benzyltoluènes, le benzylbenzène sont avantageusement éliminés dans une première distillation, en même temps que le toluène ou le xylène non réagi et recyclés avant le traitement de déchloration.

On obtient en pied une fraction lourde pouvant contenir les restes de l'agent déchlorant, du NaCl, des résidus catalytiques et des oligomères de polyarylalcane plus lourds de formule (I) et / ou (II) comme par exemple les composés de formule (I) dans laquelle n1 + n2 = 2.

Les produits obtenus peuvent être purifiés selon une technique qui consiste à employer une terre de foulon ou d'alumine activée, soit seule, soit en mélange selon les techniques spécifiques connues dans le secteur des liquides diélectriques.

De même, il peut être avantageux d'ajouter des stabilisants du type époxyde ou d'autre nature, comme par exemple le tétraphénylétain ou des antioxydants.

Ces adjuvants sont généralement ajoutés en quantités variables entre 0,001% et 10%, de préférence entre 0,01% et 0,3%.

Les compositions de l'invention sont avantageusement utilisées comme liquides diélectriques pour des isolations fonctionnant à haute température comme par exemple dans les transformateurs à puissance volumique améliorée.

Elles présentent en effet l'avantage d'avoir une tension de claquage qui augmentent avec la température contrairement aux liquides couramment utilisés tels que les huiles minérales, les huiles silicones ou les alkylbenzènes.

Ces compositions de l'invention possèdent par ailleurs une meilleur résistance aux décharges partielles. Ils présentent en outre une bien meilleure stabilité thermique.

Les compositions selon l'invention présentent également l'avantage de posséder des points d'éclair et des points de feu supérieurs à 200°C, qui est la température maximum d'utilisation.

Les exemples qui suivent illustrent l'invention.

## Exemple 1

Dans un réacteur muni d'une agitation, d'un condenseur, d'un tube d'alimentation de chlore et d'une lampe PHILIPS TLADK de 30 Watts, on charge 40 moles de toluène. On introduit 10 moles de chlore gazeux en 4 heures à la température de 90°C. Le milieu réactionnel est dégazé, refroidi puis introduit progressivement dans un réacteur contenant 4 moles de toluène et 1g de chlorure ferrique à la température 90°C. En fin de réaction, la masse réactionnelle est dégazée puis soumise à une distillation avec une colonne de quatre plateaux sous une pression réduite de 100 mm de mercure de manière à éliminer le toluène non transformé.

On abaisse ensuite la pression vers 10 mm de mercure de manière à éliminer les isomères du benzyltoluène.

La température des vapeurs est de 140°C/150°C. La température en pied augmente à 240°C. Le mélange d'oligomères obtenu en pied est constitué d'oligomères de formule (I) dans laquelle n1 + n 2 ≥ 1 avec x = 1 ainsi que des oligomères de formule (II).

Ce mélange est traité 6 heures à 300°C sous courant d'azote par 2% de méthylate de sodium puis il est soumis à une distillation sous pression réduite de 3 mm de mercure avec une colonne de 4 plateaux.

La fraction passant entre 200°C - 210°C a la composition pondérale suivante :

- 92,9% de (Ia) et (Ib)
- 4,1% d'oligomères de formule (II) dans laquelle

Z = 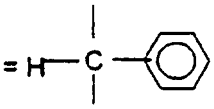

n'1 +n"1 + n'2 + n"2 + n3 + n'3 + n4 + n5 = 0 et y = z = 1 (ditolylphénylméthanes)

- 1,9% de (Ic)
- moins de 1% de benzyltoluènes (formule (I) dans laquelle n1 + n2 = 0 et x = 1)

La composition obtenue est celle qui sera utilisée dans tous les essais qui vont suivre.

♦ **Essai de détermination des tensions de claquage**.

Les mesures ont été effectuées en courant alternatif de 50 Hz en champ divergent (pointe 0,6 mm) dans une cellule telle que représentée sur la figure 1 avec pour électrodes :

⇒ un barreau de diamètre égal 0,6 mm (1)
⇒ un disque Rogowski bord arrondi, de diamètre égal à 40 mm (2) et deux volumes :

- un volume de 0,7 litre (3),
- un volume de 3 litres (4).

Pour chaque écartement entre les électrodes on a réalisé 5 à 6 mesures de la tension de claquage en appliquant une rampe de tension de 3000 V/s.

Dans une première série d'essais, nous avons effectué des mesures de tension de claquage à température ambiante (20°C), d'une part sur la composition de l'exemple 1, d'autre part sur différentes huiles minérales et sur du dodécylbenzène. Les résultats sont reportés sur le graphique 1. Sur ce graphique, nous avons représenté en ordonnée la tension de claquage en kV et en abscisse la distance entre les électrodes en mm.

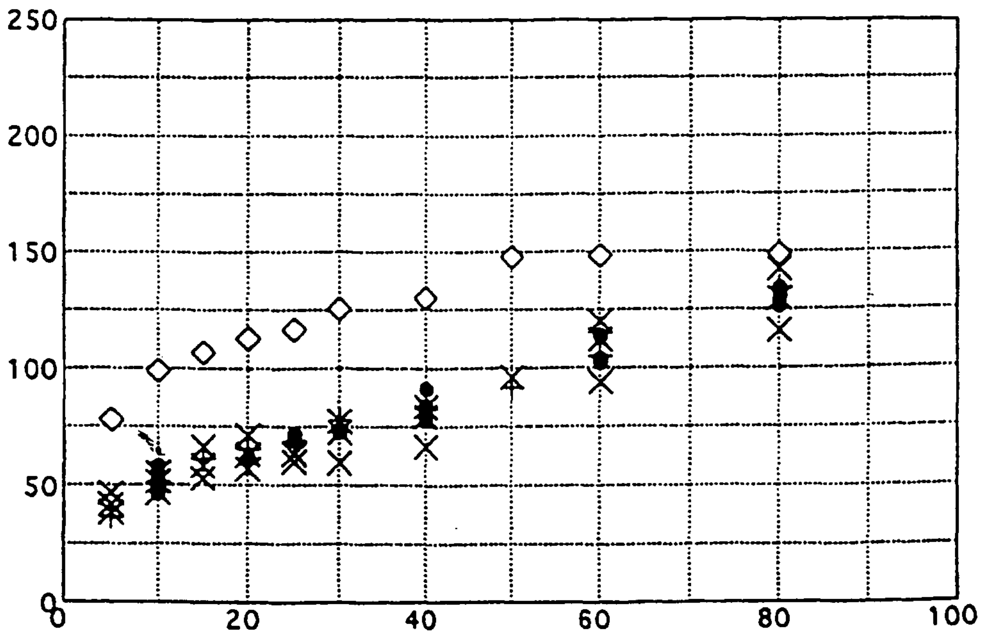


GRAPHIQUE 1

Dans ce graphique on représente également par :

● la composition selon l'exemple 1,
◇ le dodécylbenzène

X l'huile paraffinique dénommée Univolt 52,
+ l'huile minérale naphténique dénommée Nytro 10 G

On peut observer sur ce graphique que la composition selon l'exemple 1 présente systématiquement une tension de claquage égale à celle de l'huile minérale, qui augment lorsque l'écartement entre les électrodes augmente contrairement au dodécylbenzène (0) qui voit sa tension de claquage plafonner à partir d'un certain écartement.
Dans une seconde série d'essais nous avons effectué des mesures de tension de claquage à 20°C et à 80°C d'une part sur la composition de l'exemple 1, d'autre part sur du dodécylbenzène et sur l'huile minérale naphténique Nytro 10G
Les résultats sont reportés sur les graphiques 2 et 3.
Sur ces graphiques, nous avons représenté :

- en ordonnée, la tension de claquage en kV
- en abscisse, la distance entre les électrodes en mm

<u>**Sur le graphique 2**</u> - essais non conformes à l'invention, nous avons représenté par :

△ essais à 20°C }
} pour le dodécylbenzène
▲ essais à 80°C }

□ essais à 20°C }
} pour l'huile minérale naphténique Nytro 10G
■ essais à 80°C }

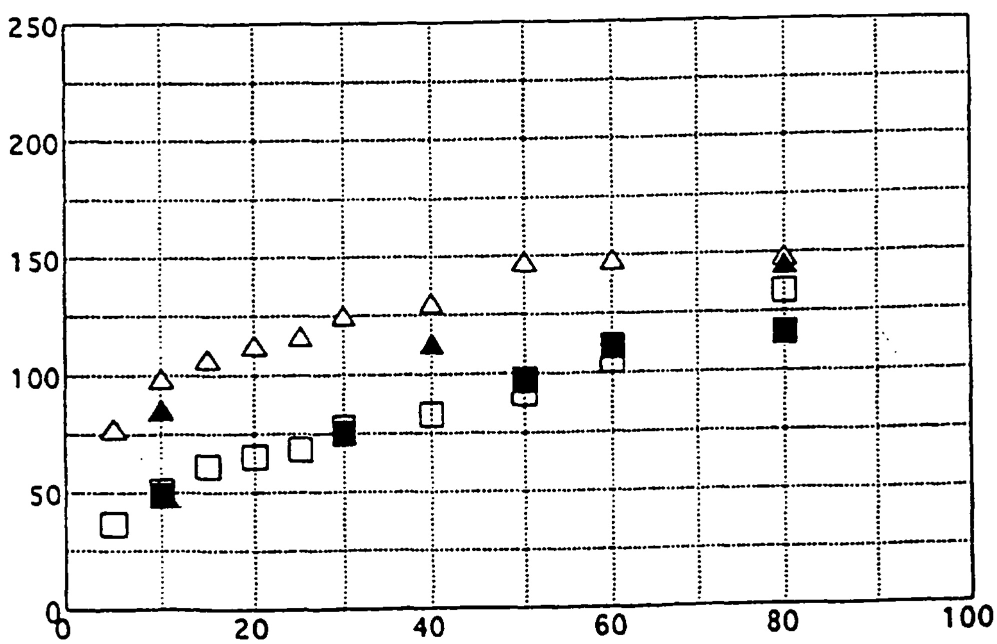


GRAPHIQUE 2

On peut observer sur ce graphique qu'il n'y a aucune différence de tension de claquage entre 20°C et 80°C, conformément à ce qui est connu.
Sur le graphique 3 (essais selon l'invention) nous avons représenté par:

□ essais à 20°C }
} sur la composition de l'exemple 1
■ essais à 80°C }

On observe une augmentation de la tension de claquage avec la température quelque soit l'écartement entre les électrodes. Par exemple, pour un écartement entre les électrodes égale à 40 mm on a une tension de claquage à 20˚C de 80 kV et à 80˚C de 130 kV.

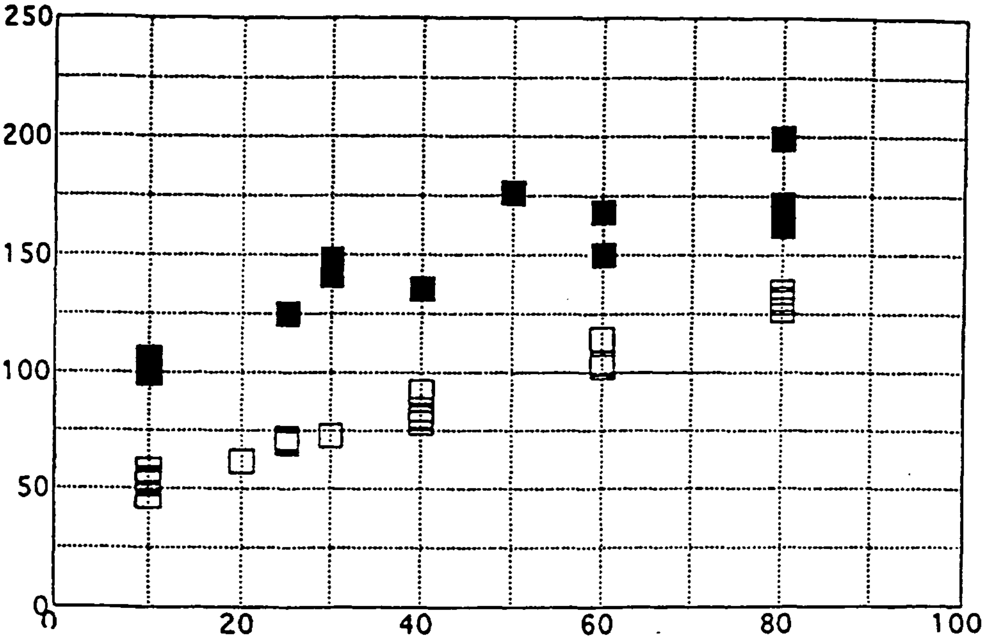


GRAPHIQUE 3

♦ **Essai de détermination de la tension de la vapeur**

Elle a été réalisée selon une méthode décrite dans :

Fluid Phase Equilibria, 42, pages 287 à 304 (1988).
Pour la composition de l'exemple 1, elle a été trouvée égale à 0,244 mm Hg à 150,9˚C

♦ **Essai de détermination de légers à 260˚C (tests de stabilité thermique)**

L'essai a été réalisé dans un appareillage tel que représenté sur la figure 2.
Les conditions de l'essai sont les suivantes :

- durée de l'essai : 500 heures
- température : 260˚C
- on récupère les produits de décomposition condensables dans un condensateur à eau et les produits de décomposition non condensables sont piégés dans une cuve à eau.

On suit la décomposition thermique au cours du temps. Les résultats sont reportés sur les graphiques 4 et 5. Sur ces graphiques nous avons représenté en ordonnée le dégagement de produits légers condensables en g/kg. (graphique 4) et en litres/kg (graphique 5) et en abscisse le temps en heures.
Nous avons également représenté par :

● l'huile paraffinique UNIVOLT 52,
■ la composition de l'exemple 1,
♦ une huile silicone utilisée dans les transformateurs,
○ un esters de pentaerythritol dénomée MIDEL.

On constate que le taux de formation de produits légers condensables et non condensables est quasiment nul à 260˚C après 500 heures.

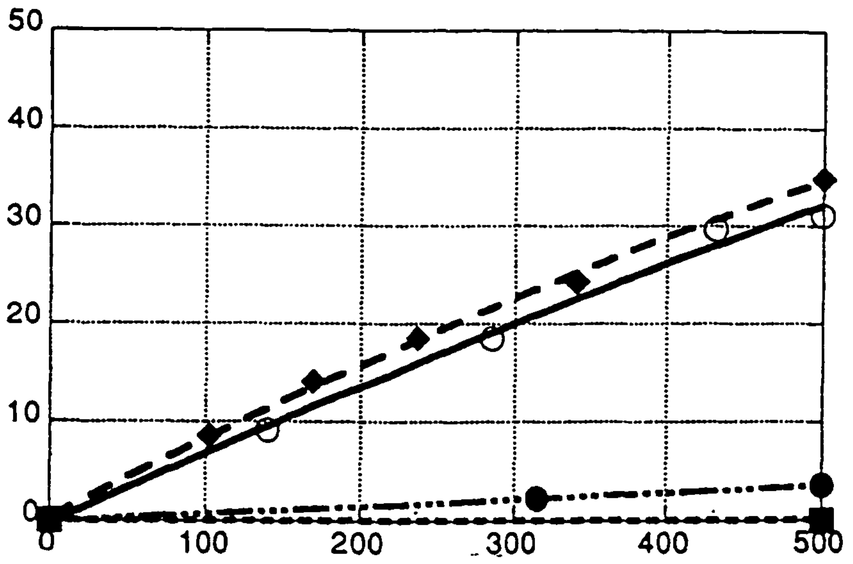


GRAPHIQUE 4

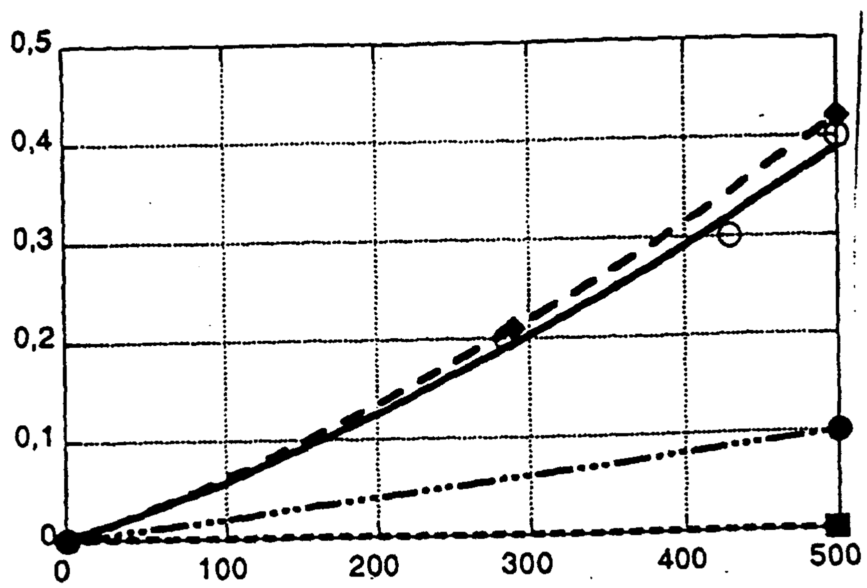


GRAPHIQUE 5

♦ **Essai de détermination du coefficient de qualité thermique**

On détermine la chaleur spécifique à 100°C par calorimétrie
On détermine la conductivité thermique à 100°C selon ASTM D 2717
On détermine la viscosité à 100°C selon ASTM D 445
Puis on applique la relation :

$$\frac{\text{chaleur spécifique X conductivité thermique}}{\text{Viscosité}} \text{ X 100}$$

Pour la composition de l'exemple 1, on a

- chaleur spécifique à 100°C = 1,802 J/l/°C
- conductivité thermique à 100°C = 0,12146 W/m/°C
- viscosité à 100°C = 3,1 $mm^2/s$

Dont le coefficient de qualité thermique est égal : $\frac{1,802 \times 0,12146}{3,1} \times 100 = 7,06$

**Revendications**

1. Utilisation pour les transformateurs de distribution dits "haute température" d'une composition diélectrique, comprenant au moins un oligomère de polyarylalcane, qui consiste en un isomère ou un mélange d'isomère de formule :

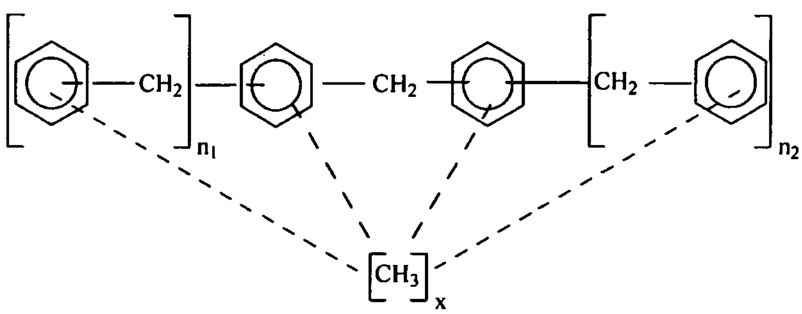

(I)

dans laquelle $n_1$ et $n_2$ = 0, 1 ou 2 sachant que $n_1 + n_2$ est égal à 1 ou 2, x = 0, 1,2,3,4, 5 ou 6 et ayant :

a) une tension de vapeur au plus égale à 133,32 Pa à 150°C,
b) un coefficient de qualité thermique défini comme le produit de la chaleur spécifique à 100°C (J/l/°C) par la conductivité thermique à 100°C (W/m/°C) divisé par la viscosité à 100°C (mm/s) et multiplié par 100 au moins égal à 3,
c) un taux de formation de légers à 260°C pendant 500 heures au plus égal à 3 g/kg, et
d) une tension de claquage à 20°C au moins égale à 60 kV, et à 80°C, au moins égal à 110 kV mesurée en courant alternatif de 50 $H_z$ dans une cellule équipée d'une électrode constituée par un barreau de diamètre égal à 0,6 mm et d'une électrode constituée par un disque Rogowski à bord arrondi de diamètre égal à 40 mm, lesdites électrodes étant séparées par une distance de 40 mm.

2. Utilisation d'une composition selon la revendication 1, **caractérisée en ce qu'**elle a :

a) une tension de vapeur au plus égale à 33,33 Pa à 150°C,
b) un coefficient de qualité thermique au moins égal à 6,
c) un taux de formation de légers à 260°C pendant 500 heures au plus égal à 1 g/kg, et
d) une tension de claquage à 20°C au moins égale à 80 kV et à 80°C, au moins égal à 130 kV.

**3.** Utilisation d'une composition selon la revendication 1 ou 2 **caractérisée en ce qu'**elle comprend une quantité pondérale d'oligomères (Ia) -oligomères de formule (I) dans laquelle $n_1 = 1$, $n_2 = 0$ et $x = 1$ et d'oligomères (Ib) -oligomères de formule (I) dans laquelle $n_1 = 0$, $n_2 = 1$ et $x = 1$ au moins égale à 40 % et une quantité pondérale d'oligomères (Ic) -oligomères de formule (I) dans laquelle $n_1$ et $n_2$ = 0, 1 ou 2 sachant que $n_1 + n_2 = 2$ et $x = 1$ - au plus égale à 3 %.

**4.** Utilisation d'une composition selon la revendication 3, **caractérisée en ce qu'**elle comprend une quantité pondérale d'oligomères (Ia) et (Ib) allant de 70 % à plus de 99 % et une quantité pondérale d'oligomères (Ic) allant de 0,5 % à 2 %.

**5.** Utilisation d'une composition selon l'une quelconque des revendications 1 à 4, **caractérisée en ce qu'**elle comprend en outre au moins un oligomère de polyarylalcane qui consiste en un isomère ou mélange d'isomères de formule :

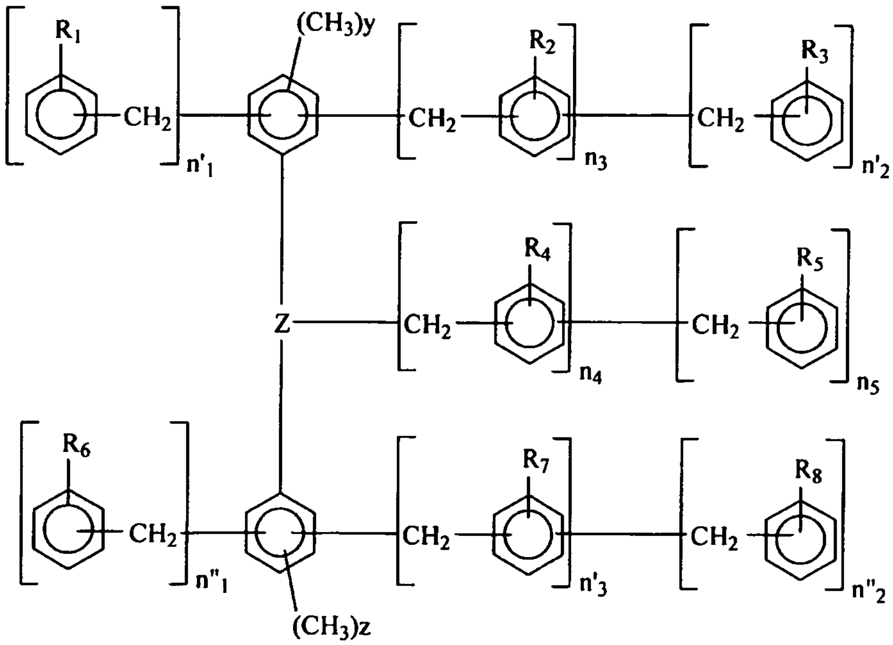

(II)

dans laquelle Z est un groupe de liaison trivalent tel que :

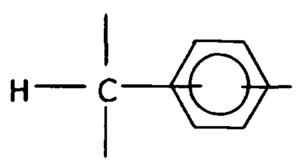

,

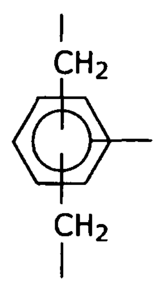

$R_1$, $R_2$, $R_3$, $R_4$, $R_5$, $R_6$, $R_7$, $R_8$ sont identiques ou différents et représentent H ou $CH_3$,
$n'_1$, $n''_1$ et $n_4$ = 0,1 ou 2,
$n'_2$, $n''_2$, $n_3$, $n'_3$ et $n_5$ = 0 ou 1,
$n'_1+n''_1+n'_2+n''_2+n_3+n'_3+n_4+n_5 \leq 2$.
y et z = 0, 1 ou 2.

**6.** Utilisation d'une composition selon la revendication 5, **caractérisée en ce que** les oligomères de formule (II) sont les ditolylphénylméthanes de formule :

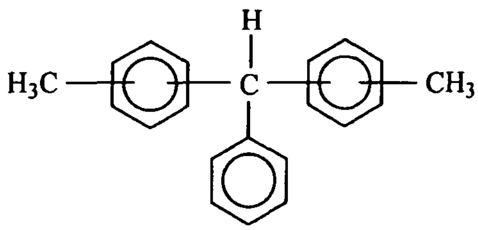

(IIa)

**7.** Utilisation d'une composition selon l'une quelconque des revendications 1 à 6, **caractérisée en ce qu'**elle comprend de :

- 60 % à 70 % en poids d'oligomères (Ia),
- 20 % à 30 % en poids d'oligomères (Ib),
- 0 % à 2 % en poids d'oligomères (Ic),
- 0 % à 10 % en poids de ditolylphénylméthanes (11a). Le total étant égal à 100 %.

## Claims

**1.** Use, for "high-temperature" distribution transformers, of a dielectric composition comprising at least one polyarylalkane oligomer, which consists of an isomer or a mixture of isomers of formula :

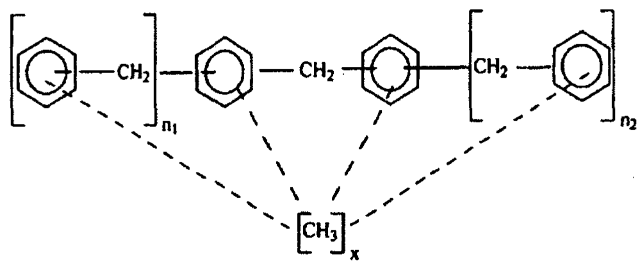

(I)

in which $n_1$ and $n_2$ = 0, 1 or 2, knowing that $n_1 + n_2$ is equal to 1 or 2, x = 0, 1, 2, 3, 4, 5 or 6, and having :

a) a vapour pressure at most equal to 133.32 Pa at 150°C;
b) a thermal quality coefficient, defined as the specific heat at 100°C (in J/1/°C) times the thermal conductivity at 100°C (in W/m/°C) divided by the viscosity at 100°C (in $mm^2/s$) and multiplied by 100, of at least 3;
c) an amount of light fractions formed after 500 hours at 260°C of at most 3 g/kg; and
d) a breakdown voltage at 20°C of at least 60 kV and at 80°C of at least 110 kV, measured under 50 Hz AC in a cell equipped with an electrode formed by a bar having a diameter of 0.6 mm and with an electrode formed by a Rogowski disc having a rounded edge and a diameter of 40 mm, said electrodes being separated by a distance of 40 mm.

**2.** Use of a composition according to Claim 1, **characterized in that** it has:

a) a vapour pressure of at most 33.33 Pa at 150°C;
b) a thermal quality coefficient of at least 6;
c) an amount of light fractions formed after 500 hours at 260°C of at most 1 g/kg; and
d) a breakdown voltage at 20°C of at least 80 kV and at 80°C of at least 130 kV.

**3.** Use of a composition according to Claim 1 or 2, **characterized in that** it comprises a quantity by weight of oligomers (Ia) - oligomers of formula (I) in which $n_1 = 1$, $n_2 = 0$ and x = 1 - and of oligomers (Ib) - oligomers of formula (I) in which $n_1 = 0$, $n_2 = 1$ and x = 1 - of at least 40% and a quantity by weight of oligomers (Ic) - oligomers of formula (I) in which $n_1$ and $n_2$ = 0, 1 or 2, knowing that $n_1 + n_2 = 2$ and x = 1 - of at most 3%.

**4.** Use of a composition according to Claim 3, **characterized in that** it comprises a quantity by weight of oligomers (Ia) and (Ib) ranging from 70% to more than 99% and a quantity by weight of oligomers (Ic) ranging from 0.5% to 2%.

**5.** Use of a composition according to any one of Claims 1 to 4, **characterized in that** it further includes at least one polyarylalkane oligomer consisting of an isomer or mixture of isomers of formula:

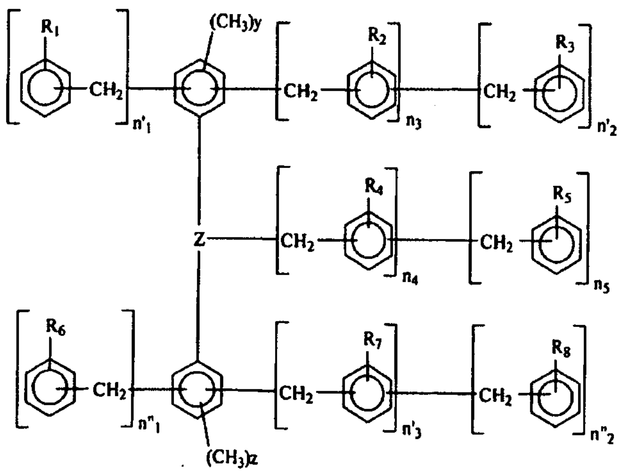

(II)

in which z is a trivalent linking group such as:

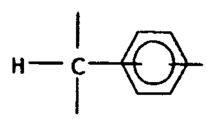

,

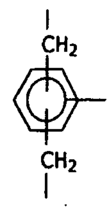

$R_1$, $R_2$, $R_3$, $R_4$, $R_5$, $R_6$, $R_7$ and $R_8$ are identical or different and represent H or $CH_3$;
$n'_1$, $n''_1$ and $n_4$ = 0, 1 or 2;
$n'_2$, $n''_2$, $n_3$, n' 3 and $n_5$ = 0 or 1;
$n'_1 + n''_1 + n'_2 + n''_2 + n_3 + n'_3 + n_4 + n_5 \leq 2$; and
y and z = 0, 1 or 2.

**6.** Use of a composition according to Claim 5, **characterized in that** the oligomers of formula (II) are ditolylphenyl-methanes of formula:

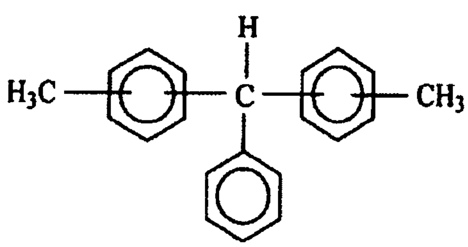

(IIa)

**7.** Use of a composition according to any one of Claims 1 to 6, **characterized in that** it comprises:

- 60% to 70% by weight of oligomers (Ia);

- 20% to 30% by weight of oligomers (Ib);
- 0% to 2% by weight of oligimers (Ic); and
- 0% to 10% by weight of ditolylphenylmethanes (IIa),

the total being equal to 100%.

**Patentansprüche**

1. Verwendung für Distributionstransformatoren, die "Hochtemperatur"-Transformatoren genannt werden, einer dielektrischen Zusammensetzung, die mindestens ein Polyarylalkanoligomer umfasst, das aus einem Isomer oder einem Isomergemisch mit folgender Formel besteht :

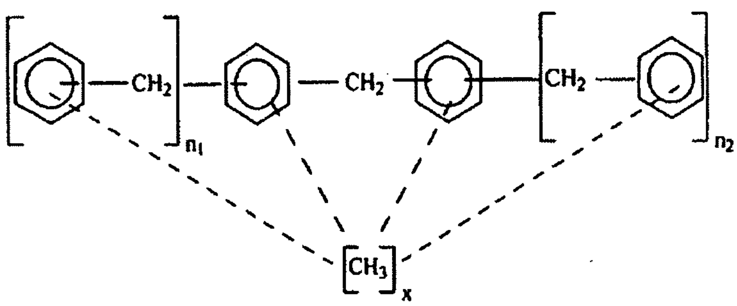

(I)

worin $n_1$ und $n_2$ = 0, 1 oder 2 sind, wobei gilt, dass $n_1 + n_2$ gleich 1 oder 2, x = 0, 1, 2, 3, 4, 5 oder 6 ist und aufweisend:

a) eine Dampfspannung von kleiner gleich 133,32 Pa bei 150˚C,
b) einen thermischen Gütekoeffizienten, definiert als das Produkt der spezifischen Wärme bei 100 ˚C (J/1/˚C) durch die Wärmeleitfähigkeit bei 100 ˚C (W/m/˚C) geteilt durch die Viskosität bei 100 ˚C ($mm^2/s$) und multipliziert mit 100 größer gleich 3,
c) eine Bildungsrate leichter Ionen bei 260 ˚C über 500 Stunden von kleiner gleich 3 g/kg, und
d) eine Durchbruchspannung bei 20 ˚C von größer gleich 60 kV, und bei 80 ˚C von größer gleich 110 kV, gemessen in Wechselstrom von 50 Hz in einer Zelle, die mit einer Elektrode ausgestattet ist, die von einem Stab mit einem Durchmesser gleich 0,6 mm gebildet wird und von einer Elektrode, die von einer Rogowski-Scheibe mit abgerundetem Rand mit einem Durchmesser gleich 40 mm gebildet wird, wobei die Elektroden durch einen Abstand von 40 mm getrennt sind.

2. Verwendung einer Zusammensetzung nach Anspruch 1, **dadurch gekennzeichnet, dass** sie Folgendes aufweist:

a) eine Dampfspannung von kleiner gleich 33,33 Pa bei 150 ˚C,
b) einen thermischen Gütekoeffizienten von größer gleich 6,
c) eine Bildungsrate leichter Ionen bei 260 ˚C über 500 Stunden kleiner gleich 1 g/kg, und
d) eine Durchbruchspannung bei 20 ˚C größer gleich 80 kV und bei 80 ˚C größer gleich 130 kV.

3. Verwendung einer Zusammensetzung nach Anspruch 1 oder 2, **dadurch gekennzeichnet, dass** sie eine Oligomergewichtsmenge (1a) -Oligomere der Formel (I) worin $n_1$ = 1, $n_2$ = 0 und x = 1 ist, und Oligomere (1b) -Oligomere der Formel (I) worin $n_1$ = 0, $n_2$ = 1 und x = 1 größer gleich 40 % und eine Oligomergewichtsmenge (1c) -Oligomere der Formel (I) worin $n_1$ und $n_2$ = 0, 1 oder 2 sind, wobei gilt, dass $n_1 + n_2$ = 2 und x = 1 - kleiner gleich 3 %, umfasst.

4. Verwendung einer Zusammensetzung nach Anspruch 3, **dadurch gekennzeichnet, dass** sie eine Oligomergewichtsmenge (Ia) und (1b), die von 70 % bis zu mehr als 99 % reicht und eine Oligomergewichtsmenge (1c), die von 0,5 % bis 2 % reicht, umfasst.

5. Verwendung einer Zusammensetzung nach irgendeinem der Ansprüche 1 bis 4, **dadurch gekennzeichnet, dass** sie zudem mindestens ein Polyarylalkanoligomer umfasst, das aus einem Isomer oder einem Isomergemisch der folgenden Formel besteht:

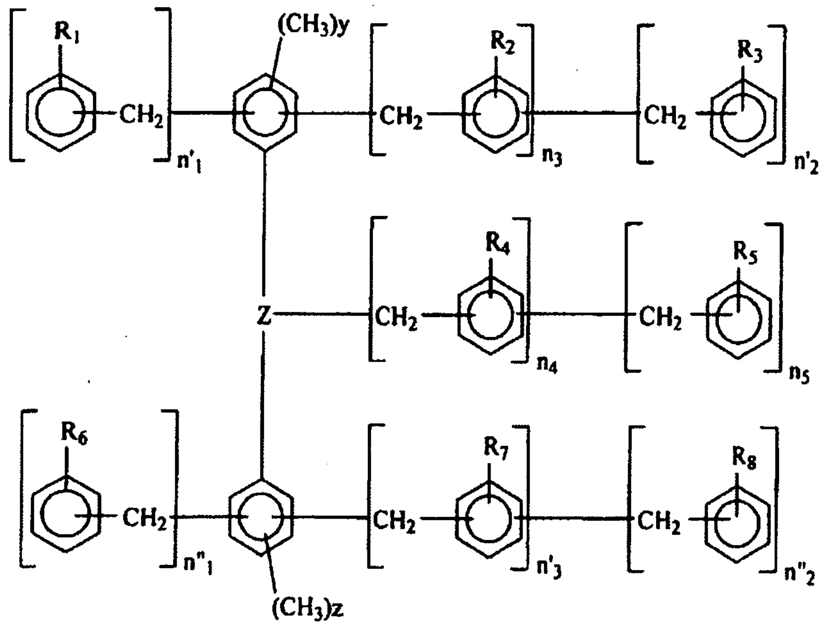

(II)

worin Z eine dreiwertige Verbindungsgruppe ist, wie etwa:

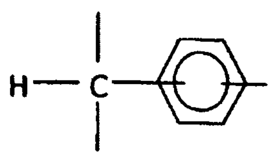

,

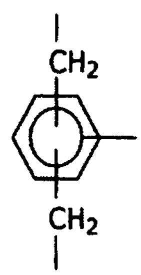

worin $R_1$, $R_2$, $R_3$, $R_4$, $R_5$, $R_6$, $R_7$, $R_8$ gleich oder verschieden sind, und H oder $CH_3$ darstellen,
$n'\ 1$, $n''_1$ und $n_4$ = 0, 1 oder 2 sind,
$n'_2$, $n''_2$, $n_3$, $n'_3$ und $n_5$ = 0 oder 1 sind,
$n'_1 + n''_1 + n'_2 + n''_2 + n_3 + n'_3 + n_4 + n_5 \leq 2$ ist,
y und z = 0, 1 oder 2 sind.

**6.** Verwendung einer Zusammensetzung nach Anspruch 5, **dadurch gekennzeichnet, dass** die Oligomere der Formel (II) Ditolylphenylmethane der folgenden Formel sind:

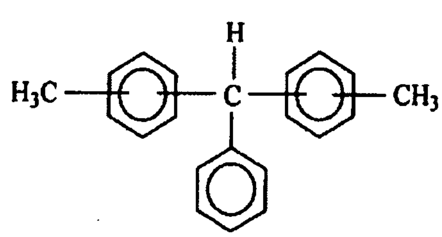

(IIa)

**7.** Verwendung einer Zusammensetzung nach irgendeinem der Ansprüche 1 bis 6, **dadurch gekennzeichnet, dass** sie umfasst:

- 60 Gew.-% bis 70 Gew.-% Oligomere (1a).
- 20 Gew.-% bis 30 Gew.-% Oligomere (1b).
- 0 Gew.-% bis 2 Gew.-% Oligomere (1c).
- 0 Gew.-% bis 10 Gew.-% Ditolylphenylmethane (IIa),

wobei der Gesamtwert gleich 100 % ist.

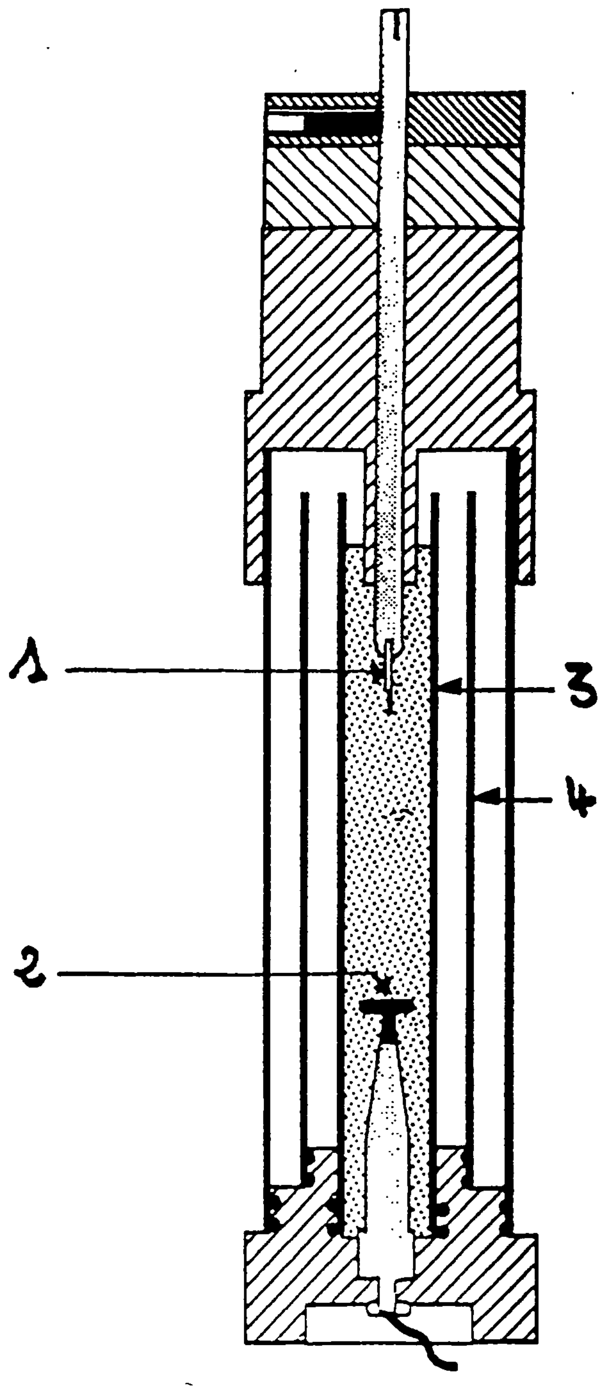


FIGURE 1

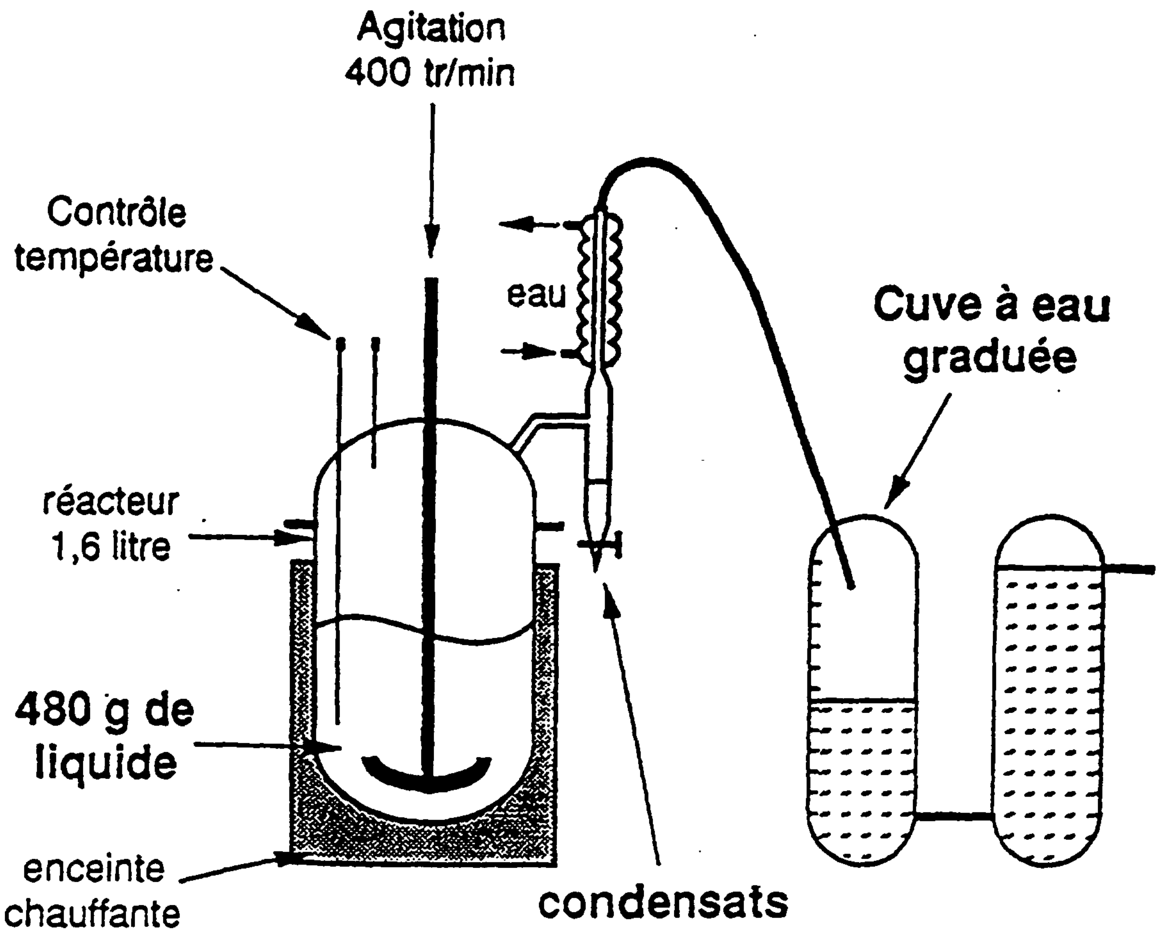
Agitation
400 tr/min
Contrôle
température
eau
Cuve à eau
graduée
réacteur
1,6 litre
480 g de
liquide
enceinte
chauffante
condensats

FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 306398 A
- EP 225849 A


### Littérature non-brevet citée dans la description


- *rapport HCP/T-2115,* Février 1979, 35
- *IEEE Trans. Electr. Insul,* Août 1978, vol. El - 13, 263
- **FRANCK M. CLARK.** Insulating Materials for Design and Pratice. John Wiley and Sons, Inc, 151
- *Fluid Phase Equilibria,* 1988, vol. 42, 287-304